# EUROPEAN PATENT APPLICATION

(11) **EP 3 718 511 A1**
(43) Date of publication of application: **07.10.2020**
(21) Application number: 19167201.3
(22) Date of filing: 04.04.2019
(51) Int. Cl.: A61F 2/34, A61F 2/32

(54) **RESURFACING CUP FOR ACETABULUM HEMIARTHROPLASTY OF THE HIP JOINT**

(71) Applicant: Scyon Orthopaedics AG, 8005 Zürich (CH)
(72) Inventor: Tepic, Slobodan, CH-8005 Zurich (CH)
(74) Representative: Weickmann & Weickmann PartmbB

(57) **Abstract**

The present invention disclosed resolves the main problems of bone-sparing hip resurfacing surgery by using only an acetabular component in which the femoral head articulates, being only shaved to a spherical shape and cleared of osteophytes that could impinge on the acetabulum and so restrict the range of motion. The inner surface of the resurfacing cup (10) is aspherical in shape creating an annular contact with the femoral head. The inner, articulating surface of the cup is ADLC or pyrolytic carbon coated to reduce friction and wear of the femoral head. The surgical approach is well developed with surgery time significantly reduced by avoiding the use of the femoral resurfacing component. The cup is preferably of the double-shell type for cementless fixation, but it can also be made as a single shell for cementless or cemented fixation. Should a revision surgery due to wear of the femoral head become necessary, the cup can be retained and combined with a dual mobility femoral component.

## Description

The present invention relates to an acetabular cup for hemiarthroplasty of the hip joint, for use in human and veterinary medicine. Further, the present invention relates to a dual mobility revision total hip replacement of the acetabular resurfacing cup and to a femoral head shaver to prepare a femoral head for articulation inside the above acetabular resurfacing cup.

### Background

Hip replacement surgery is considered one of the most successful, quality of life improving surgical procedures of the past century. The history of hip surgery in broader use started in the late forties with Austin-Moore, a hemi-prosthesis for the femoral head and neck. The femoral stem was cementless, with fenestrations for bone ingrowth.

The first total hip prostheses were of the metal-on-metal type, such as the Thompson hemi-prosthesis modified by McKee, introduced in the fifties. Clinical results were mixed and the acceptance was limited.

The revolution in clinical acceptance and the dawn of modern total hip replacement is attributed to Charnley who in mid-sixties introduced a cemented stem and a cemented polymer cup. That basic concept is still in broad clinical use in spite of all the advancements in cementless fixation of both the femoral and acetabular components.

In the past two decades, most of the development in total hip arthroplasty was focused on articulation in order to reduce wear of components, mostly of the polymeric cups. State of the art today is the combination of a ceramic head on the femoral side and a cross-linked, vitamin-E supplemented, Ultra-High-Molecular-Weight Polyethylene (UHMWPE) acetabular cup. Acetabular cups are either provided with metal backing for bony integration or are cemented.

Another approach to hip arthroplasty is by a so-called resurfacing procedure that came into clinical use in two waves, both of which ended up in failures of the concept. The first attempts in mid-seventies by Wagner and Amstutz have not found a successful following. The latest resurfacing approach is due to McMinn with his Birmingham model of a resurfacing total hip replacement based on metal-on-metal articulation. Excessive wear of metallic components, however, led to major clinical complications and spelled the end to this second wave of bone-sparing hip replacements.

Hemiarthroplasty is still commonly used, but for various reasons, more common today is a so-called bi-polar model, where the conventional femoral component is combined with a large diameter head that articulates with the intact acetabulum. This model for obvious reasons is limited to use in patients with an acceptable level of degeneration of the acetabular cartilage - usually for femoral neck fractures in old patients.

In summary, all of the above prior hip prostheses comprised a femoral component of some sort, paired to either the intact acetabulum or to an acetabular cup.

In the last decades of hip replacement surgery, there was an increasing need for its use in young and active patients. Wear of polymeric acetabular cups turned out to be a limiting factor in the durability of the prosthesis. At typically 7 to 9 years after surgery, accumulation of wear particles in and around the joint would lead to bone loss and aseptic loosening of components. While in average patients of 70 years of age at the time of primary replacement the revision rate at 15 years post-op is below 5%, in young and active patients it is 20 to 25%. The most direct reason for the difference is the level of activity - wear of polymer in all lab tests is proportional to the number of cycles.

Clinical outcomes have improved with newer, wear-resistant articulations, such as ceramic heads on cross-linked UHMWPE cup inlays, but the problem of aseptic loosening in young and active patients remains significant leading to high revision burden - in some countries as high as one in five. Secondary procedures last even shorter.

Use of resurfacing total hip replacement was mainly motivated by sparing the femoral bone mass so that eventual revision by a stemmed femoral component would be easier to perform. As the metal-on-metal design of resurfacing prosthesis failed due to metal wear causing so-called "tumor-like" tissue response, this option has basically been lost and is performed by only a handful of surgeons in the world mostly for very young patients involved in high-demand sports.

Hemi-prostheses are currently used only in cases where acetabulum cartilage is still in good condition. A patent application by the present inventor (US 2013/0060345A1) discloses a potential improvement on the current practice by proposing an aspherical pairing of a hemi-prosthesis, wherein the prosthesis head is covered by either amorphous-diamond-like-carbon (ADLC) coating or by pyrolytic carbon. These carbon coatings have been shown to dramatically reduce wear of the opposing bone when compared to metals or ceramics. Aspheric articulations, such as described in the US Patent 8,323,346 by Tepic, have reduced wear in lab testing by approximately three times.

The hip replacement proposed according to the present invention is of a novel type where the only prosthetic component is the acetabular cup. The acetabular cup hemi-prosthesis is combined with a native femoral head. The femoral head remains almost intact - it may only be shaved into a substantially spherical shape and irregularities, such as osteophytes around the head are removed to reduce the risk of impingement.

### Summary of the Invention

The present invention resolves the main problems of bone-sparing hip resurfacing surgery by using only an acetabular component in which a native femoral head articulates, being only shaved to a spherical shape and cleared of osteophytes that could impinge on the acetabulum and so restrict the range of motion. Preferably, the inner surface of the resurfacing cup is aspherical in shape creating an annular contact with the femoral head. The inner, articulating surface of the cup is preferably coated with a carbon-based material, such as ADLC and/or pyrolytic carbon to reduce friction and wear of the femoral head. The surgical approach is well developed with surgery time significantly reduced by avoiding the use of the femoral resurfacing component. The cup is preferably of the double-shell type for cementless fixation, but it can also be made as a single shell for cementless or cemented fixation. Should a revision surgery due to wear of the femoral head become necessary, the cup can be retained and combined with a dual mobility femoral component.

Thus, a first aspect of the present invention is an acetabular resurfacing cup for hemiarthroplasty of the hip joint. The acetabular resurfacing cup is adapted to a native femoral head, i.e. a femoral head having a native bone surface. The acetabular cup of the present invention is particularly adapted for human medicine, but it may also be used in veterinary medicine, e.g. for dogs.

The inner surface of the acetabular resurfacing cup is facing the femoral head. The shape of this inner surface may be aspherical and may comprise an annular section which is of spherical shape. The angle covered by the annular spherical section is preferably in the range of 5-30°, more preferably in the range of 10-20° The angle from the axis of symmetry of the acetabular resurfacing cup to the annular spherical section is preferably in the range of 25-50°, more preferably 30-45° The axis of symmetry of the inner shape of the cup may be offset from the main axis of symmetry of the cup by an angle preferably in the range of 5-30°, more preferably in the range of 15-25°.

The inner surface of the cup may be coated by any suitable material, e.g. by a carbon-based material such as ADLC and/or pyrolytic carbon.

The cup of the present invention may be adapted for cementless fixation, e.g. by a double shell construct or as a single shell. On the other hand, the cup may be also adapted for cemented fixation.

A further aspect of the present invention relates to a hip prosthesis consisting of an acetabular resurfacing cup, as described above.

Still a further aspect of the present invention relates to a femoral head shaver for preparing the femoral head of a patient for articulation, particularly for articulation inside an acetabular resurfacing cup, as described above. The femoral head shaver may comprise a shaving cup covering about a hemisphere with an inner surface of over about 180 degrees included angle, wherein the inner surface comprises cutting protrusions, e.g. cutting flutes, for reaming bone, and openings for removing of bone chips. The shaving cup is adapted for a rotating and swiveling operation, e.g. by being connected to a rod or a similar element.

Still a further aspect of the present invention relates to a combination of a femoral head shaver and an acetabular resurfacing cup, as described above.

Still a further aspect of the present invention relates to a method for hemiarthroplasty of the hip joint, comprising implanting an acetabular resurfacing cup, as described above.

Still a further aspect of the present invention relates to a combination of an acetabular resurfacing cup, as described above and an artificial femoral component, e.g. a femoral head for dual mobility revision total hip replacement.

Still a further aspect of the present invention relates to a method for dual mobility revision total hip replacement of an acetabular resurfacing cup, as described above.

In preferred embodiments of the present invention, the articulations are aspherical and have carbon-based coatings providing a novel combination of features, wherein the acetabulum and only the acetabulum is resurfaced by a prosthetic component and the femoral head is kept in its basically native condition.

There are several important advantages of this combination. It requires a very small amount of bone to be removed to start with. If a revision were required, it would be only of the femoral side using a dual mobility type femoral replacement where the primary prosthetic head is covered by a polymeric cup, which then articulates against the resurfaced acetabulum. Reaming of the acetabulum is also minimal since the cup may only be about 1 to about 4 mm thick, preferably about 1.5 to about 3 mm thick. Reaming of the acetabulum is generally associated with much lower risks than reaming of the femoral cavity known to lead to some very serious complications such as pulmonary embolism. In press-fitted stems, there is also a high risk of bone fissures. In the Birmingham type resurfacing total prostheses, the femoral head is reamed to a particular shape and then covered by a cemented cup with a centering pin. Bone loss is smaller than in conventional stemmed femoral components, but the blood supply of the remaining femoral head bone is compromised and can lead to massive bone necrosis. In contrast, in the present invention, the head is only cleaned of major irregularities and shaved into a spherical shape to fit into the resurfaced acetabulum. Preferably, the acetabular cup cavity is aspherical and covered by a carbon-based material coating, e.g. ADLC coating or by pyrolytic carbon. The time of surgery is significantly reduced and so is the cost of the prosthesis. The surgical approach is well known and has been used by many surgeons for Birmingham-type resurfacing.

### List of figures

- Fig. 1: shows a hip replacement total-prosthesis according to the state of the art.
- Fig. 2: shows a hip replacement hemi-prosthesis according to the state of the art.
- Fig. 3: shows a resurfacing hip replacement prosthesis according to the state of the art.
- Fig. 4: shows a hemi replacement prosthesis of the hip joint according to the invention.
- Fig. 5: shows a double shell acetabular hemi hip replacement prosthesis according to the invention.
- Fig. 6: shows a single shell acetabular hemi replacement prosthesis according to the invention.
- Fig. 7: shows a femoral head shaving instrument according to the invention.
- Fig. 8: shows a revision surgery with a dual mobility femoral head component.

### Detailed description

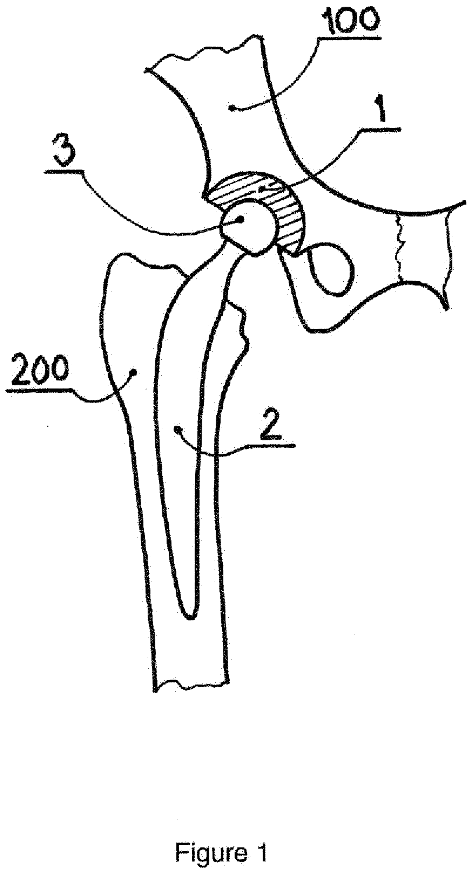

A state of the art total hip replacement prosthesis, Figure 1, comprises an acetabulum cup 1 fixed to the pelvis 100, a femoral stem 2 fixed to the femur 200 with the femoral head 3 attached to the stem 2. A typical configuration today has a polymeric cup inlay, most commonly from a cross-linked UHMWPE, backed by a metal shell with a porous coating facing the bone. The stem for cementless fixation may be coated with a porous layer too, or alternatively rough-blasted with a thin coating of, for example, hydroxyapatite. The head can be metallic or ceramic. In an alternative to cementless fixation, both the cup and the stem can be cemented into respective bones using so-called bone cement, a two-component poly-methyl-methacrylate.

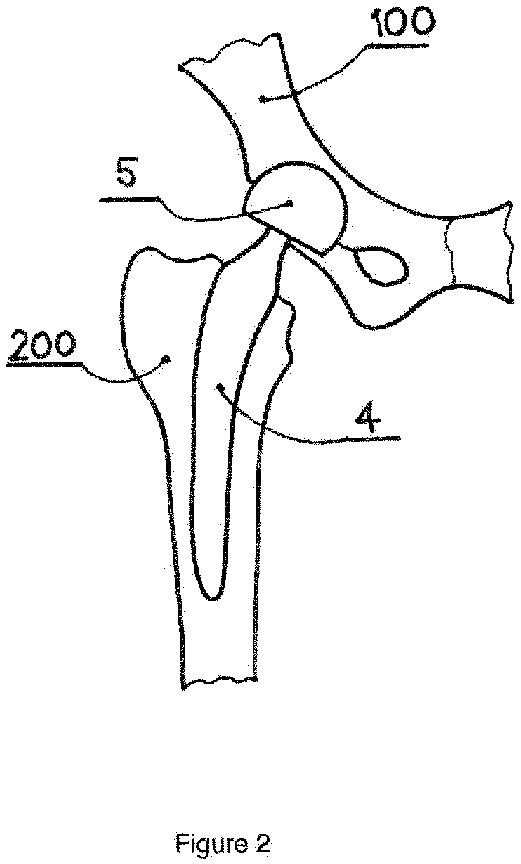

A state of the art hemi hip replacement prosthesis, Figure 2, comprises only a femoral component. A femoral stem 4 is fixed to the femur 200. A large diameter femoral head 5 is fitted to the stem 4 either directly, or via a smaller head to form a bi-polar hemi-prosthesis.

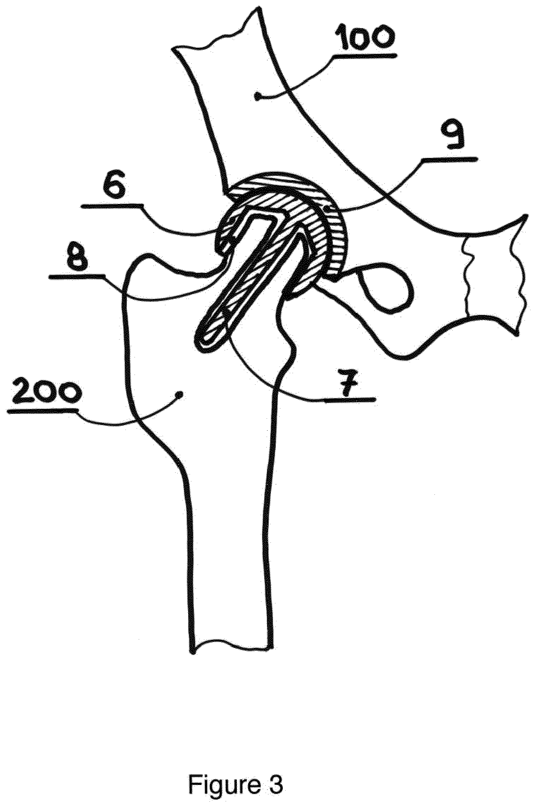

A Birmingham-type resurfacing total hip prosthesis, Figure 3, comprises a femoral component 6 with a large diameter head, usually cemented to cap the femoral head, reamed down to accommodate the prosthetic head and the centering pin 7 with some space allowed to be filled with bone cement 8. The acetabular component 9 is usually of cementless type, hammered into the reamed acetabulum 100.

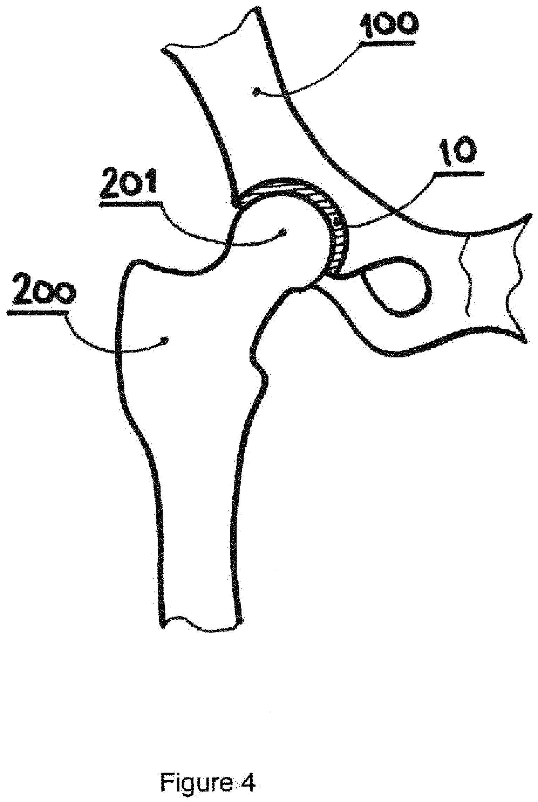

A hip replacement hemi-prosthesis according to this invention, shown in Figure 4, spares most of the femur 200 and relies on only an acetabulum resurfacing cup 10, fixed into the pelvis 100. The size of the cup 10 is selected to fit over the native femoral head 201, which is reamed into a spherical shape by removing as little cartilage and bone as possible to create a spherical shape, removing all gross deformities in form of osteophytes which tend to form at the neck of the femur. The cup is preferably of cementless type, but it could also be cemented into the reamed-out acetabulum.

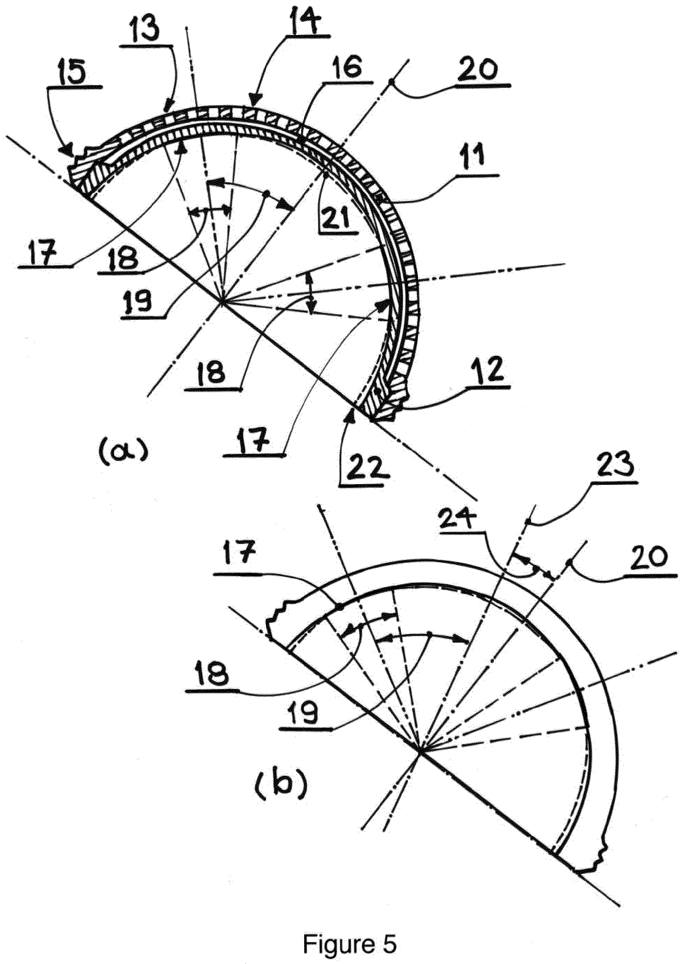

A double shell acetabular cup according to the invention, shown in Figure 5a, comprises an outer shell 11 and an inner shell 12. A double shell acetabular cup for a total hip replacement is disclosed in US Patent 7,776,097 by Tepic and Malchau. The cup may comprise perforations 13 in the outer shell to allow for a rapid invasion by bone. The outside, bone-facing surface of the shell 11, may be coated by porous material such as titanium and hydroxyapatite 14 thereby providing additional micro fixation. The press-fit stability of the cup in the reamed-out acetabulum may be enhanced by small extensions such as ribs 15 in the outer shell close to the opening of the cup. The inner shell 12 may be fitted, e.g. snap-press fitted into the outer shell 11 at the opening of the cup leaving a small gap 16 between the shells. The size of the gap is usually about 0.5 to about 1.5 mm.

This combination results in a highly compliant metal construct for integration into the pelvic bone without stress shielding observed when thick, stiff metal backing is used for conventional total hip cups. Preferably, the inner shell of the cup is from harder and/or stronger material than the outer shell. In certain embodiments, the tensile strength of the metal forming the outer shell is about 300 to about 600 MPa, preferably from about 350 MPa to about 550 MPa and/or the hardness on the Vickers scale (Hv) of the metal forming the outer shell is about 100 to about 250 Hv, preferably from about 120 to about 200 Hv. In certain embodiments, the tensile strength of the metal forming the inner shell is about 700 to about 1,000 MPa, preferably about 800 to about 950 MPa, more preferably about 900 MPa, and/or the hardness on the Vickers scale (Hv) of the metal forming the inner shell is about 280 to about 450 Hv, preferably about 300 to about 400 Hv, more preferably about 350 Hv.

Preferred metals for the shells are titanium and titanium alloys. For example, the outer shell could be produced from a commercially pure (c.p.) titanium grade 2, or preferably grade 4. Alternatively, a titanium-aluminum-niobium alloy can be used for its higher strength but also superb biocompatibility. The inner shell needs to be stronger and harder than is possible with c.p. grades of titanium, so an alloy like titanium-aluminum-niobium (Ti6AI7Nb) and/or possibly titanium-aluminum-vanadium (Ti6Al4V) are preferred choices. Titanium c.p. grades' tensile strength is in the range of 350 to 550 MPa; hardness in the range of 120 to 200 on the Vickers scale (Hv). Corresponding values for the Ti6Al7Nb and Ti6Al4V alloys are 900 MPa and 350 Hv.

The inner shell is of aspherical shape, providing the general advantages presented in the US Patent 8,323,346 by Tepic. The inner surface 17, over an annular band covering an angle 18 of 5 to 30 degrees, more preferably 10 to 20 degrees is spherical in shape. It is offset from axis 20 by an angle 19 of 25 to 50 degrees, more preferably 30 to 45 degrees. The section of the cup between the band 17 and the pole is shaped so as to create a gap 21 with respect to the spherical shape of the annulus 17. From section 17 out towards the opening of the cup, the shape of the inner shell is also deviating from a sphere creating a gap 22. The aspherical shape of the inner surface of the cup aids in lubrication and lowers the friction and wear of the articulation.

To further reduce friction and wear of the femoral head articulating in the replacement cup, the inner surface of the inner shell is coated by a suitable material, e.g. by ADLC or by pyrolytic carbon.

For the optimal function of the aspherical articulation the axis of symmetry of the inner surface shape 23, should be offset with respect to the axis of symmetry 20 of the whole cup as shown in Figure 5b. The angle 24 of 5 to 30 degrees, more preferably of 15 to 25 degrees would bring the axis 23 into the middle of the range of the joint force in gait. When implanting into the pelvis the offset 24 should be placed in the superior direction of the pelvis.

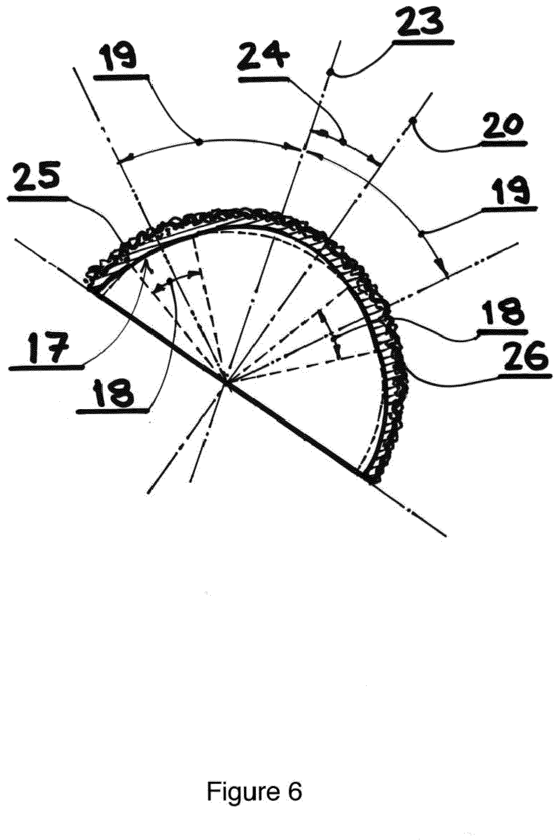

An alternative make-up of the acetabular cup for hemi hip replacement is shown in Figure 6. A single shell 25 with an inner shape as disclosed for the cup of Figure 5 is either coated on the outside by a porous layer 26 for cementless bony ingrowth, or is adapted for cemented fixation into a reamed-out acetabulum in which case the outer surface is usually only textured or provided by other such means for improved interface to the bone cement. Cups of this type may be produced by either conventional machining or additive manufacturing. Preferred metals for cementless fixation are titanium alloys. For cemented type cup, cobalt-chromium alloys can also be used and the choice here can also be expanded to some stainless steels suitable for ADLC coating.

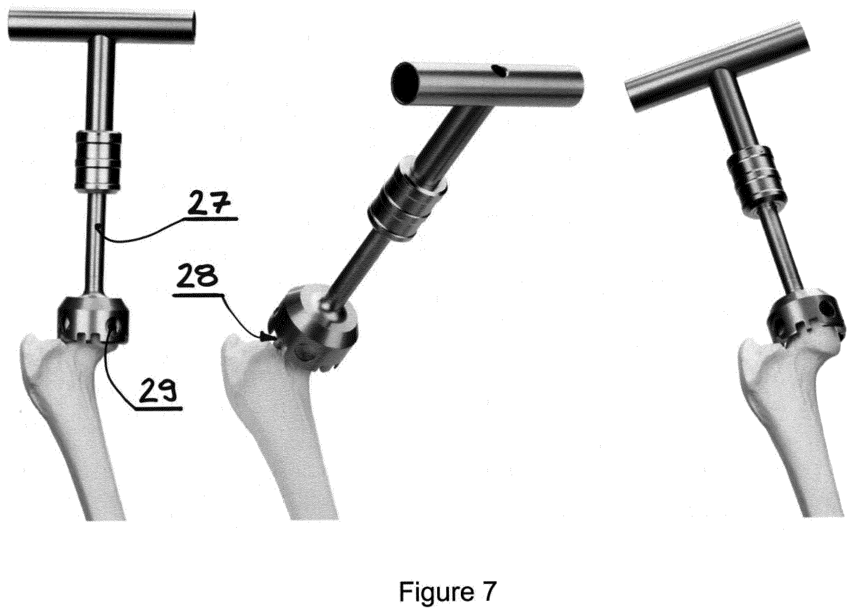

An instrument 27 to shave the femoral head into a spherical shape is shown on Figure 7. It should be used with caution and preferably only by hand. The reamer 27 covers about a hemisphere, i.e. the inner surface of the reamer over about 180 degrees included angle is covered by cutting flutes 28. Openings 29 in the reamer allow for bone chips to be removed during and after reaming of the femoral head.

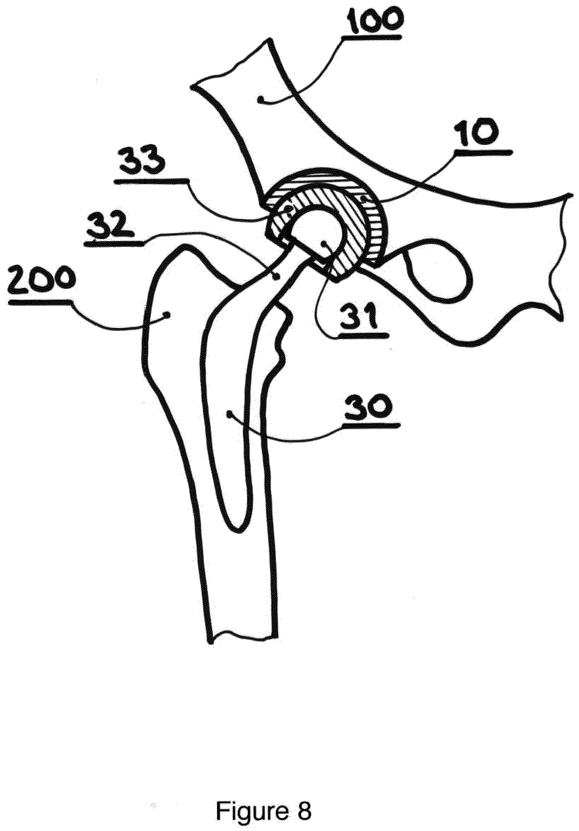

If a revision surgery would become necessary, the cup 10 of the present invention as described above can be retained and only a femoral component can be inserted, Figure 8. The primary head 31 seated on the femoral neck 32 of the stem 30, is snap-fitted with a secondary head 33, made out of a polymer, such as UHMWPE or poly-ether-ether-ketone (PEEK). This secondary head can now be articulated inside the cup 10.

A partial hip without the cup disclosed in patent application US 2013/0060345A by Tepic, has been used in hundreds of dogs for over 8 years without any indication of the heads penetrating into the pelvis. In some cases, the dogs revert to full function of the hip in a matter of weeks albeit somewhat slower than with a total hip replacement. In other cases, recovery has been slower, taking several months to reach a steady state. It is unclear at this time what might slow down the recovery, but it safe to say that this is tied to the process of bone remodeling around the joint. As the reaming of the acetabulum is always done to the maximum depth without penetrating the medial wall of the acetabulum, the bone exposed to the articulation will vary a lot - from a dense subchondral bone to a soft cancellous bone and possibly some remnants of the cartilage covered areas.

In the procedure disclosed in this invention, the reaming of the femoral head will be less aggressive and in many cases, the bone just under the articulating surface is dense and sclerotic, possibly devoid of any pain receptors. This should cut the recovery time in addition to cutting the time of surgery and hence contribute to an increase of the all-important benefit-to-cost ratio.

## Claims

1. An acetabular resurfacing cup 10 for hemiarthroplasty of the hip joint.

2. An acetabular resurfacing cup 10 of claim 1, wherein the shape of the inner surface facing the femoral head 201, is of aspherical shape.

3. An acetabular resurfacing cup 10 of claim 2, wherein there is an annular section 17 of the inner surface which is of spherical shape.

4. An acetabular resurfacing cup 10 of claim 3, wherein the angle 18 covered by the annular spherical section 17 is in the range of 10 to 20 degrees.

5. An acetabular resurfacing cup 10 of claim 3 or 4, wherein the angle 19 from the axis of symmetry 20 to the annular spherical section 17 is in the range of 30 to 45 degrees.

6. An acetabular resurfacing cup 10 of any one of claims 3-5, wherein the axis of symmetry 23 of the inner shape of the cup is offset from the main axis of symmetry 20 of the cup by an angle 24 in the range of 15 to 25 degrees.

7. An acetabular resurfacing cup 10 of any one of claims 1-6, wherein the inner surface of the cup facing the femoral head is coated by a carbon-based material such as ADLC and/or pyrolytic carbon.

8. An acetabular resurfacing cup 10 of any one of claims 1-7, adapted for cementless fixation.

9. An acetabular resurfacing cup 10 of claim 8, adapted for cementless fixation which is a double shell construct 11/12.

10. An acetabular resurfacing cup 10 of claim 8, adapted for cementless fixation which is a single shell construct 25.

11. An acetabular resurfacing cup 10 of any one of claims 1-7, adapted for cemented fixation.

12. A hip prosthesis consisting of an acetabular resurfacing cup according to any one of claims 1-11.

13. A femoral head shaver 27 for preparing a femoral head for articulation, particularly for articulation inside an acetabular resurfacing cup 10 of any one of claims 1-11.

14. A combination of an acetabular resurfacing cup 10 of any one of claims 1-11 and an artificial femoral component, e.g. a femoral head adapted for dual mobility revision total hip replacement 30/31/33.
